# EUROPÄISCHE PATENTANMELDUNG

(11) **EP 3 378 399 A1**
(43) Veröffentlichungstag der Anmeldung: **26.09.2018**
(21) Anmeldenummer: 17162900.9
(22) Anmeldetag: 24.03.2017
(51) Int. Cl.: A61B 6/00, G01N 23/20, G01V 5/00

(54) **RÖNTGENSTREUMESSVORRICHTUNG UND VERFAHREN ZUR RÖNTGENSTREUMESSUNG**

(71) Anmelder: Universität zu Lübeck, 23538 Lübeck (DE)
(72) Erfinder: Harding, Geoffrey, 22547 Hamburg (DE); Isernhagen, Fabian, 23558 Lübeck (DE); Buzug, Thorsten M., 23627 Gross Sarau (DE)
(74) Vertreter: RCD Patent Giesen, Schmelcher & Griebel Patentwanwälte PartG mbB

(57) **Zusammenfassung**

Die Erfindung betrifft eine Röntgenstreumessvorrichtung aufweisend einen Tunnel (30) zur Aufnahme eines Objekts und einen vorbestimmten Durchstrahlungsbereich im Innern des Tunnels (30), der mit einer Mehrzahl von Fächern aus Röntgen-Fingerstrahlen durchsetzbar ist, sowie eine in den Tunnel (30) einbringbare Auflageplattform (60) für das Objekt mit wenigstens einem Antriebsmittel zur kontrollierten Bewegung der Auflageplattform (60) im Tunnel (30), dadurch gekennzeichnet, dass die Auflageplattform (60) mit dem wenigstens einen Antriebsmittel zur Bewegung entlang der Tunnelachse und entlang wenigstens einer Achse senkrecht zur Tunnelachse ausgebildet ist.

## Beschreibung

Die Erfindung betrifft eine Röntgenstreumessvorrichtung und ein Verfahren zur Röntgenstreumessung.

Die Erfindung betrifft insbesondere eine bildgebende Röntgenstreuvorrichtung zur Analyse und Lokalisierung chemischer Substanzen im Inneren eines Objekts, wobei die Röntgenstreuvorrichtung einen Tunnel zur Aufnahme des Objekts und einen vorbestimmten Durchstrahlungsbereich im Innern des Tunnels, die mit einer Mehrzahl von Fächern aus Röntgen-Fingerstrahlen durchsetzbar ist, aufweist. Die Erfindung betrifft ferner ein Verfahren zur Röntgenstreumessung eines Objekts, insbesondere auch eines lebenden Patienten für medizinische Zwecke.

### Hintergrund

Gattungsgemäße Vorrichtungen sind als sogenannte "Gepäck-Scanner" für Sicherheitsbereiche, insbesondere an Flughäfen, bekannt und kommerziell erhältlich. Zum einschlägigen Stand der Technik wird beispielsweise auf die Druckschriften US 6,054,712 oder auch EP 2 221847 B1 und die dort zitierten Referenzen verwiesen. Es wird darauf hingewiesen, dass die in EP 2 221 847 B1 beschriebene Technik auch als X-ray Diffraction Imaging (XDI) bezeichnet wird.

Der Durchstrahlungsbereich einer solchen Röntgenstreuvorrichtung ist eine Querschnittsfläche des Tunnels, die von polychromatischem Röntgenlicht durchsetzt wird. Beispielsweise emittiert eine oberhalb des Tunnels angeordnete Röntgenanode das Röntgenlicht, das durch den Durchstrahlungsbereich propagiert und von einem unterhalb des Tunnels angeordneten Detektor-Array erfasst wird. Das Detektor-Array umfasst dabei eine Mehrzahl energieintegrierend messender Transmissionsdetektoren angeordnet entlang des unteren Randes des Durchstrahlungsbereichs sowie zu jedem Transmissionsdetektor eine benachbart angeordnete, entlang der Tunnelachse ausgerichtete lineare Anordnung von energieaufgelöst messenden Streulichtdetektoren. Die Streulichtdetektoren können nur dann ein Signal erfassen, wenn ein im Durchstrahlungsbereich vorliegendes Objekt einen Anteil des Röntgenlichts um kleine Winkel kohärent streut oder beugt.

Wenn man für die einzelnen Volumenelemente - i. F. Voxel - eines Objekts die Amplitude, d.h. die Signalstärke am Detektor, in Abhängigkeit der Photonenenergie und des Streuwinkels kennt, kann man aus diesen Daten ein Impulsübertrag-Profil ("momentum transfer profile") ermitteln, das als charakteristisch für das streuende Material anzusehen ist, also gewissermaßen einen "Fingerabdruck" der streuenden Substanz oder Zusammensetzung darstellt. Dazu ist sicherzustellen, dass jeder Streudetektor zu jedem Zeitpunkt nur die Streustrahlung eines einzelnen Voxels erfasst und dass überdies die dreidimensionale Position des Voxels im Raum bestimmt wird. Dies kann beides zugleich durch Kollimatoren erreicht werden.

In einem multiplen Fächerstrahl ("multiple fan-beam", MFB) Aufbau wird die Röntgenstrahlung durch eine senkrecht zur Tunnelachse und zur Lotrichtung (= Schwerkraftrichtung) orientierte, lineare Anordnung von gleichmäßig beabstandeten, einzeln aktivierbaren Röntgenanoden erzeugt. Ein zwischen den Röntgenanoden und dem Tunnel angeordneter Primärkollimator ist so ausgebildet, dass er bei Aktivierung jeweils einer einzelnen Röntgenanode der linearen Anordnung einen Fächer von Fingerstrahlen in den Tunnel eintreten lässt, wobei jeder einzelne Fingerstrahl auf genau einen Konvergenzpunkt gerichtet ist, an dem sich einer der Transmissionsdetektoren befindet. Auf diese Weise erzeugt jede einzelne aktivierte Röntgenanode einen gegenüber den emittierbaren Fächern der anderen inaktiven Röntgenanoden versetzt verlaufenden Fächer von Fingerstrahlen, wobei alle Fächer in derselben Querschnittsfläche des Tunnels liegen und jeder Voxel im Durchstrahlungsbereich von höchstens einem der Fingerstrahlen durchstrahlt wird. Werden die Röntgenanoden jeweils einzeln nacheinander aktiviert, wird durch das Durchschalten der zueinander versetzten Röntgenfächer erreicht, dass jeder Voxel des Tunnels im Durchstrahlungsbereich irgendwann von einem der Fingerstrahlen durchsetzt ist. Durch die MFB Anordnung wird jeder Voxel im Durchstrahlungsbereich von durchschnittlich drei unterschiedlichen Einstrahlrichtungen her mit Strahlung durchsetzt. Dadurch wird Information über die isotropen bzw. anisotropen Eigenschaften des Voxels gewonnen.

Auf der anderen Seite beschränkt ein Sekundärkollimator, der zwischen Tunnel und Detektor-Array angeordnet ist, die Raumwinkel, aus denen die Detektoren Strahlung erfassen können. Dadurch "sieht" insbesondere jeder Streulichtdetektor nur eine begrenzte Anzahl von Voxeln in einem vorbestimmten Segment und in einer vorbestimmten Höhenlage des Durchstrahlungsbereichs. Zu einer Detektorerfassung von Streustrahlung kann es nur kommen, wenn sich ein streuendes Objektelement in einem der für den Detektor sichtbaren Voxel befindet, dabei von einem der Fingerstrahlen durchsetzt wird und ein Streuprozess stattfindet. Wenn der Detektor ein Signal erfasst, dann ergibt sich aus dem Aktivierungszustand der Röntgenanoden bei der Messung, welcher der Voxel des Durchstrahlungsbereichs den Streulichtbeitrag geleistet hat.

Bewegt man ein zu untersuchendes Objekt mit angemessener Geschwindigkeit durch den Durchstrahlungsbereich der Messvorrichtung im Tunnel, dann korrespondieren die Voxel des Durchstrahlungsbereichs zu vorbestimmten Zeiten mit den Voxeln des Objekts in einem Objektkoordinatensystem und lassen sich entsprechend zuordnen.

Es kann dann in einem Rechner beispielsweise eine Tabelle erzeugt werden, die die Messdaten der Detektoren als Funktionen der Voxel-Position in Objektkoordinaten und der Photonenenergie enthält. In Anbetracht der vorbekannten Positionen der Detektoren sind zugleich die Streuwinkel der Datenerfassung bekannt. Die vorgenannte tabellierte skalarwertige Funktion über einem 4-dimensionalen Intervall lässt sich umrechnen in eine Tabelle aus Impulsübertrags-Profilen, die jeweils einzeln einem Voxel des Objekts zugeordnet sind. Diese Tabelle wird im Folgenden kurz als 4D-Datensatz bezeichnet.

Da die Impulsübertrags-Profile zur Klassifikation oder Identifikation von chemischen Substanzen herangezogen werden können, ist mit den Messdaten der Vorrichtung eine Substanz(klassen)zuweisung auf die Voxel des zu untersuchenden Objekts möglich, was zugleich eine dreidimensionale Darstellung des Befundes in einem Objektmodell ermöglicht. In diesem Sinne ist die Röntgenstreuvorrichtung bildgebend in Analogie zur bekannten Computer-Tomographie.

Die vorbeschriebene Scanner-Technologie wird derzeit zur schnellen Durchsuchung von Gepäckstücken nach gefährlichen oder illegalen Substanzen, insbesondere zur Detektion von Explosivstoffen, eingesetzt. Das Gepäck wird dabei üblich auf einem Transportband mit konstanter Geschwindigkeit durch den Tunnel bewegt. Erkennt das System eine der potentiell gefährlichen Substanzen, wird ein Alarm ausgelöst und in der Regel auch die Unterstützung eines Mitarbeiters des Sicherheitspersonals angefordert. Dies führt zu Zeitverzögerungen und ist kostenträchtig. Zu den wichtigen Zielen der technischen Entwicklung in diesen Anwendungen zählt daher - neben der sicheren Erkennung der gesuchten Substanzen als Hauptziel - auch die Vermeidung von Fehlalarmen.

Nach Kenntnis der Erfinder wurde die medizinische Verwendung eines derartigen Scanners zur Bildgebung der inneren Organe eines lebenden Patienten bislang noch nicht ernsthaft verfolgt. Dabei liegen einige Vorteile eines medizinischen Scanners nach diesem Prinzip auf der Hand:
- Man kann Information über die molekulare Struktur lebenden Gewebes gewinnen, die auf keine andere Weise in einem solchen Umfang erzeugt und erfasst werden kann.
- Es gibt bekannte Zusammenhänge zwischen dem Ausbruch einer Krankheit und der vorherigen Änderung der molekularen Zusammensetzung des Gewebes. Die Röntgenstreudaten können daher Aufschluss geben in Bezug auf - ggf. beginnende - Erkrankungen oder Verschleißerscheinungen des menschlichen Körpers.
- Es wird eine Röntgendosis für einen Volumenscan appliziert, die etwa zwei Größenordnungen geringer als die Dosis eines herkömmlichen CT-Scans desselben Volumens ist.

Zudem sind die Messdauern relativ kurz und die technischen Komponenten zur Messung praktisch alle fertig entwickelt und kommerziell erhältlich.

Auf der Seite der Nachteile des Standes der Technik für medizinische Zwecke stehen aber die geringe Auflösung der Messung und die bislang ungeklärte Anschlussfähigkeit von Daten, die mit Zeitabstand nacheinander erfasst werden.

Die geringe Auflösung ergibt sich aus den in heutigen Scannern üblichen Durchmessern der Voxel in der Größenordnung Zentimeter. Dies mag für das Schnellscreening von Gepäck völlig ausreichen, wo es um das Auffinden nicht nur sehr kleiner Mengen von illegalem Material geht. Die Voxel sind jedoch zu groß zur Bilderzeugung von medizinisch wichtigen Details. Zur Verbesserung könnte man zwar eine größere Anzahl von Röntgenanoden, dichtere Röntgendetektor-Arrays und komplexer strukturierte Kollimatoren als bisher üblich in Betracht ziehen, doch wären die Mehrkosten beachtlich, und man stieße auch bald an technische Grenzen.

Die Anschlussfähigkeit zeitlich versetzter Messungen bezeichnet die Möglichkeit, Messdaten desselben Objekts, die nacheinander - ggf. mit einem Zeitabstand von Tagen oder Wochen - erfasst worden sind, in einen Bezug zueinander zu setzen und nach Bedarf in der Zusammenschau auszuwerten.

Bei bekannten Gepäckscannern stellt sich die Frage der Anschlussfähigkeit nicht, weil es ohnehin praktisch sehr selten vorkommt, dass dasselbe Objekt, z.B. ein gepackter Koffer, mehrfach gescannt wird (der Kofferinhalt unterscheidet sich und ist auch jeweils anders verteilt). Zudem sind Bezugnahmen zwischen Scans zu verschiedenen Zeiten in dieser Anwendung auch uninteressant.

In der medizinischen Anwendung ist das durchleuchtete Objekt hingegen ein lebender Patient, der in der Regel nicht nur einmal, sondern regelmäßig wiederholt untersucht wird, etwa um Verlaufskontrollen einer Therapie oder auch Vorsorgeuntersuchungen durchzuführen. Die Anschlussfähigkeit der Scandaten ist hierbei ganz offenkundig von sehr großer Bedeutung.

Da der erzeugte 4D-Datensatz aber nur Impulsübertrags-Profile umfasst, die auf chemische Zusammensetzungen hin analysierbar sind, ist es bislang nicht geklärt, wie man die Messwerte der Patientenanatomie so zuordnen kann, dass chemische Veränderungen zwischen den Zeitpunkten der Messung als solche auch erkannt und zugeordnet werden können. Der Patient, der zur Messung durch den Tunnel der Vorrichtung bewegt werden muss, könnte bei den verschiedenen Messungen unterschiedlich auf dem Patientenbett gelegen oder auch Körpergewicht zu- oder abgenommen haben. Die korrekte Zuweisung der ortsfesten Voxel des Durchstrahlungsbereichs auf die körperbezogenen Voxel des Patienten ist wichtig für die Dateninterpretation und bedarf einer geeigneten Referenzierung auch bei großen Zeitabständen der Messungen, um Veränderungen der Körperchemie als solche zu erkennen.

Die Erfindung stellt sich die Aufgabe, eine bildgebende Röntgenstreuvorrichtung so fortzubilden, dass sie mit kostengünstigen Mitteln sowohl zur Erhöhung der Auflösung als auch zur Anschlussfähigkeit zeitlich versetzt erfasster Messdaten desselben Objekts ertüchtigt ist.

### Kurzdarrstellung der Erfindung

Die Aufgabe wird gelöst durch eine Röntgenstreumessvorrichtung aufweisend einen Tunnel zur Aufnahme eines Objekts und einen vorbestimmten Durchstrahlungsbereich im Innern des Tunnels, der mit einer Mehrzahl von Fächern aus Röntgen-Fingerstrahlen durchsetzbar ist, sowie eine in den Tunnel einbringbare Auflageplattform für das Objekt mit wenigstens einem Antriebsmittel zur kontrollierten Bewegung der Auflageplattform im Tunnel, dadurch gekennzeichnet, dass die Auflageplattform mit dem wenigstens einen Antriebsmittel zur Bewegung entlang der Tunnelachse und entlang wenigstens einer Achse senkrecht zur Tunnelachse ausgebildet ist.

Die Unteransprüche geben vorteilhafte Ausgestaltungen der Vorrichtung und Verfahrensschritte zur Durchführung medizinischer Bildgebung mit der Vorrichtung an.

Die erfindungsgemäße Vorrichtung ermöglicht es zunächst, einen auf der Auflageplattform, z.B. eine Patientenliege, liegenden Patienten in an sich bekannter Weise entlang der Tunnelachse, i. F. auch als Z-Achse bezeichnet, über eine vorbestimmte Vorschubstrecke durch den im Tunnel fest eingerichteten Durchstrahlungsbereich ganz oder teilweise hindurchzubewegen und dabei einen ersten 4D-Datensatz zu erfassen. Weiterhin gestattet die Vorrichtung das Verschieben der Auflageplattform und des Patienten entlang einer Achse, die senkrecht zur Tunnelachse gerichtet ist, um eine vorbestimmte Versatzstrecke. Die nunmehr versetzte Auflageplattform kann dann erneut über die Vorschubstrecke entlang der Z-Achse bewegt werden, wobei ein zweiter 4D-Datensatz erfasst werden kann. Liegt der Patient während der drei genannten Bewegungen ruhig und möglichst bewegungslos, so liegt der Unterschied zwischen dem ersten und zweiten erfassten 4D-Datensatz in der Versatzstrecke begründet, die zu einer jeweils unterschiedlichen "Materialbelegung" der Voxel des Durchstrahlungsbereichs während der Messungen unter Bewegung über die Vorschubstrecke geführt hat.

Die Voxel sind Volumenelemente mit in der Regel mehreren Kubikzentimetern Volumen. Beispielsweise kann ein Voxel ein Quader mit Volumen dV = dX * dY * dZ sein, wobei die Voxel-Kantenlängen senkrecht zur Tunnelachse, dX und dY, durch Anzahl und Anordnung von Röntgenanoden und Detektor-Array in Verbindung mit den Kollimatoren vorbestimmt sind, wie eingangs erläutert.

Die Voxel-Kantenlänge dZ wird a priori auch durch den apparativen Aufbau nahe gelegt, nämlich durch den entlang der Z-Achse vorliegenden Durchmesser ΔZ der Röntgen-Fingerstrahlen. Sie hängt zudem von der Vorschubgeschwindigkeit V_{P} ab, mit der der Patient durch den Durchstrahlungsbereich hindurch bewegt wird. Ist die Messdauer zur Erfassung eines Röntgenstreumesswertes für einen Voxel zu T_{M} vorbestimmt, so kann man dZ = V_{P} * T_{M} als Definition der Kantenlänge vorsehen. Für eine zweckmäßig lückenlose Durchstrahlung des Patienten bei gleichzeitig guter Auflösung und geringer Strahlenbelastung kann man auch alternativ dZ = ΔZ setzen und die Vorschubgeschwindigkeit zu V_{P} = ΔZ / T_{M} einrichten.

Es ist für Bildgebungszwecke grundsätzlich vorteilhaft, wenn sich die Voxel-Kantenlängen dX, dY und dZ möglichst wenig voneinander unterscheiden.

Der Durchmesser der Fingerstrahlen in Z-Richtung, ΔZ, kann u.a. dadurch vergrößert werden, dass der Durchstrahlungsbereich eine gegen die Lotrichtung in Richtung der Tunnelachse geneigte Ebene ist. Eine solche Neigung der Ebene, in der alle Fingerstrahlen verlaufen, ist eine vorteilhafte Ausgestaltung, da dies zu längeren Lichtwegen im Patientengewebe und in der Folge zu größeren Streuquerschnitten und einem stärkeren Messsignal führt.

Die von den Detektoren erfassten Röntgenstreumesswerte sind über einen Voxel integrierte Werte, die Streubeiträge von sehr verschiedenen Substanzen, hier: Gewebearten, umfassen können, welche in enger Nachbarschaft zueinander liegen. Ein einzelner, während der Bewegung über die Vorschubstrecke erfasster 4D-Datensatz beschreibt insofern ein 3D-Modell der chemischen Zusammensetzung des Patienten, das - in Analogie zu einem mit geringer Ortsauflösung erfassten Foto - als erheblich unscharf oder auch "verpixelt" beschrieben werden kann. Es werden in beiden Fällen zu viele Objektinformationen auf die integrierenden Detektoren abgebildet, wodurch Ortszuweisung teilweise verloren geht.

Die erfindungsgemäße Vorrichtung kann zwei oder mehr 4D-Datensätze erfassen, die sich aufgrund der "Verpixelung" voneinander unterscheiden. Diese 4D-Datensätze stehen in einer bekannten Beziehung zueinander aufgrund der vorbestimmten Translation des Patienten, die zwischen den Datenerfassungen erfolgt ist.

Vorzugsweise ist die Länge der Versatzstrecke zwischen zwei Datenerfassungen, kleiner als die Voxel-Kantenlänge entlang der Versatzrichtung vorbestimmt. Sie kann vorzugsweise ein ganzzahliger Bruchteil der Voxel-Kantenlänge sein, z.B. die Hälfte. In diesem Fall gehen Röntgenstreumessdaten aus den korrespondierenden Voxeln der Mehrzahl von erfassten 4D-Datensätzen anteilig aus denselben Gewebestrukturen des Patienten hervor, weil die korrespondierenden Voxel entsprechend der kurzen Versatzstrecke einander überlappen. Dies bietet Gelegenheit, die Mehrzahl von 4D-Datensätzen in der Zusammenschau auszuwerten und mittels eines Superresolutions-Verfahrens einen neuen, auf kleinere Voxel verfeinerten, d.h. höher ortsaufgelösten 4D-Datensatz zu errechnen.

Aus der Erhöhung der Ortsauflösung der 4D-Datensätze ergibt sich dann die Möglichkeit, im Patienten erkannte Gewebezusammensetzungen präziser zu lokalisieren, so dass anatomische Strukturen klarer erkennbar werden. Dies gestattet wiederum das Referenzieren von 4D-Datensätzen anhand der Patientenanatomie und somit die Anschlussfähigkeit zeitversetzter Messdatenerfassungen.

### Kurzdarstellung der Figuren

Die Erfindung wird im Folgenden näher erläutert auch anhand von Figuren. Dabei zeigt:
- Fig. 1: eine Schnittskizze durch den Durchstrahlungsbereich einer Röntgenstreumessvorrichtung mit multiplen Fächerstrahlen (Stand der Technik);
- Fig. 2a: und 2bSkizzen der erfindungsgemäßen Auflageplattform in der Ausgestaltung als Patientenbett a) in der perspektivischen Ansicht und b) in der Aufsicht;
- Fig. 3: eine Skizze wie in Fig. 1 mit einer trapezförmigen Ausgestaltung des Tunnelquerschnitts speziell vorgesehen für die Patientenmessung;
- Fig. 4: eine Skizze zum Vorgehen eines Super-Resolutions-Verfahren.

### Ausführliche Darstellung derErfindung

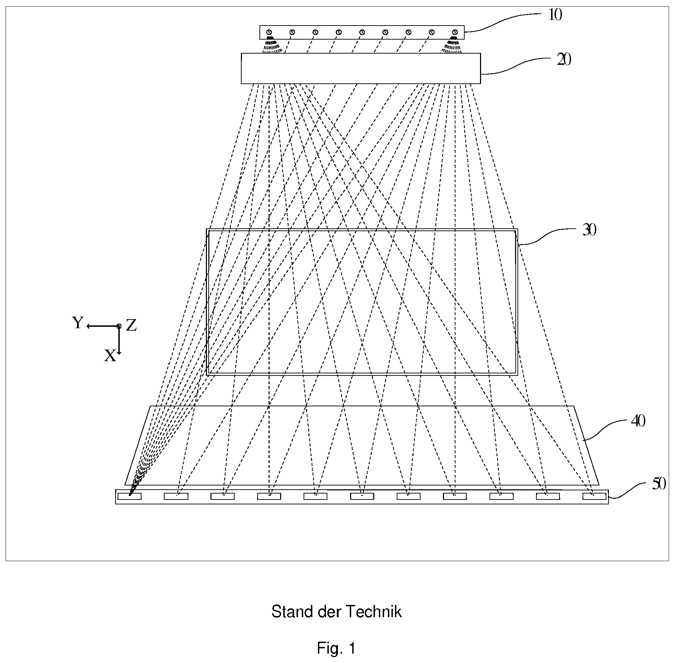

In der Fig. 1 ist ein Querschnitt durch eine Röntgenstreumessvorrichtung skizziert, wie sie aus dem Stand der Technik als Gepäck-Scanner bekannt ist. Eine Anordnung von Röntgenanoden 10 und ein Primärkollimator 20 sind so angeordnet, dass bei Aktivierung einer einzelnen Röntgenanode ein Fächer von Fingerstrahlen - für die zwei äußersten Anoden exemplarisch eingezeichnet - in den Tunnel 30 eingestrahlt wird. Jeder der Fingerstrahlen ist auf einen der Transmissionsdetektoren des Detektor-Arrays 50 gerichtet, wobei er den Sekundärkollimator 40 unterhalb des Tunnels 30 passieren muss. Die Zeichenebene fällt in diesem Beispiel mit dem Durchstrahlungsbereich im Innern des Tunnels 30 zusammen, und die Tunnelachse steht senkrecht zur Zeichenebene, wie das Dreibein links unten andeutet. Die Streulichtsensoren des Detektor-Arrays 50 sind nicht dargestellt; sie liegen in der Zeichnung vor oder hinter den Transmissionsdetektoren verteilt entlang der Tunnelachse. Die Röntgenanoden der Anordnung 10 sind einzeln aktivierbar, so dass bei sequenzieller Aktivierung jeweils ein anderer, versetzt liegender Fächer von Fingerstrahlen eingestrahlt wird.

Der gesamte Querschnitt des Tunnels 30 wird während einer Aktivierungssequenz der Anodenanordnung 10, bei der jede Anode mindestens einmal aktiviert wird, von Fingerstrahlen durchsetzt. Es gibt somit keinen Voxel im Durchstrahlungsbereich, der vom Röntgenlicht nie durchsetzt würde und schon deshalb kein Röntgenstreumesssignal erzeugen könnte. Anders ausgedrückt, jeder Voxel im Durchstrahlungsbereich muss messaktiv sein, d.h. einen Beitrag zur Streustrahlung leisten können.

Der Tunnel 30 muss stets ein ausreichend großes Volumen, das auch den Durchstrahlungsbereich umfasst, möglichst vollständig umschließen, d.h. insbesondere die geschlossenen Seitenwände des Tunnels 30 sind unverzichtbar. Die Röntgenstreumessung könnte sonst durch von außen einfallende Strahlung empfindlich gestört werden oder die Daten könnten durch mehrfach gestreute Photonen verfälscht werden, was sich durch eine abschirmende Auskleidung der Wände des Tunnels 30 z.B. mit einem Hartschaum wirksam verhindern lässt.

Bei Gepäck-Scannern ist die Auflageplattform mit wenigstens einem Antriebsmittel zum Bewegen von Objekten durch den Durchstrahlungsbereich im Innern des Tunnels 30 üblicherweise ein Förderband. Es ist dabei nur die Bewegung entlang der Vorschubrichtung des Förderbandes vorgesehen. Für die Untersuchung lebender Patienten wird man auf eine Patientenliege zurückgreifen, die nun erfindungsgemäß wenigstens einen zusätzlichen Bewegungsfreiheitsgrad aufweisen soll.

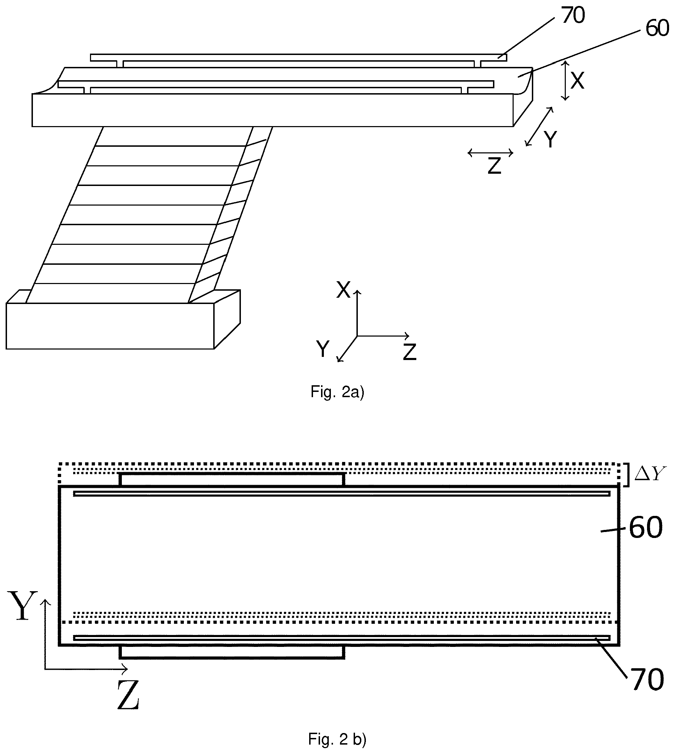

In Fig. 2 a) ist eine perspektivische Ansicht einer möglichen Ausgestaltung der Auflageplattform 60 zu sehen. Die Auflageplattform 60 ist vorzugsweise rechteckig ausgebildet, wie die Aufsicht in Fig. 2b zeigt, wobei die langen Seiten entlang der Vorschubrichtung (Z-Achse) zum Bewegen des Patienten durch den Tunnel 30 ausgerichtet sein sollen. An den langen Seiten sind vorzugsweise Seitengeländer 70, besonders bevorzugt aus einem Metall wie Stahl oder Aluminium oder auch aus einem Kunststoffkomposit, angeordnet, die zwei Zwecken dienen:
(i) der Patient soll dem Rand der Auflageplattform 60 nicht zu nahe kommen, also durch die Geländer 70 begrenzt werden, und
(ii) die Geländer 70 erzeugen eigene, eindeutig patientenfremde Streusignale in den 4D-Datensätzen, die die Auswertung unterstützen können.

Hierfür kann das vorgenannte Kunststoffkomposit beispielsweise aus einem Duroplast mit einem Füllstoff gebildet sein. Der Füllstoff soll dabei die Eigenschaft aufweisen, bei Röntgenbestrahlung ein Streusignal zu erzeugen, das sich von Streusignalen des Patienten, sowohl des Gewebes als auch etwaig vorhandener Implantate, leicht unterscheiden lässt. Beispielsweise kommen Carbon Nanotubes oder andere Partikel mit Kohlenstoff-Kohlenstoff-Bindungen als geeignete Füllstoffe in Frage. Anders gesagt soll das Kunststoffkomposit eine vom Patientenkörper unterscheidbare Röntgenstreusignatur aufweisen.

Wie in der Zeichnung angedeutet, sind Seitwärtsverschiebungen der Auflageplattform 60 um Versatzstrecken ΔX oder ΔY erfindungsgemäß vorgesehen, wobei die in Fig. 2b) gezeigte Versatzstrecke ΔY zur Verdeutlichung mehrere Breiten der Seitengeländer 70 beträgt. In der Regel werden die Versatzstrecken aber klein sein, in der typischen Größenordnung einiger Millimeter bis zu wenigen Zentimetern, vorzugsweise kleiner als die Voxel-Kantenlänge im Durchstrahlungsbereich, wie zuvor erläutert.

Aus Fig. 2a) ist weiterhin zu entnehmen, dass die Auflageplattform (60) auf ihrer Oberseite muldenförmig ausgebildet ist, d.h. sie wird entlang der Y-Richtung von den Seiten zur Mitte hin dünner. Dies wird als vorteilhaft für die Patientenmessung angesehen, da man bei Röntgenstreumessungen eine möglichst gleichmäßige Abschwächung der Röntgenstrahlen über die Breite des Detektor-Arrays 50 anstrebt. Eine muldenförmige Patientenliege 60 fügt dem zu den Seiten hin weniger massiven Patientenkörper durch zusätzliche Polsterung am Rand weiteres Material zur Röntgenabschwächung hinzu.

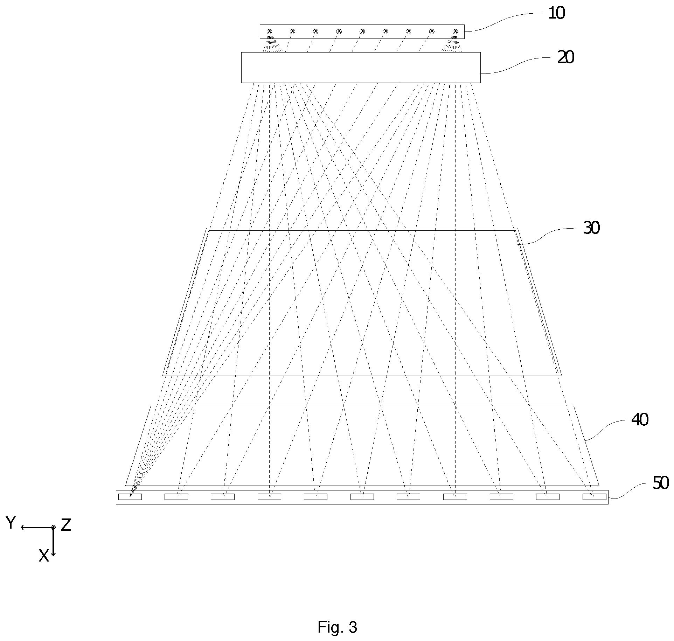

Eine weitere vorteilhafte Ausgestaltung der zur medizinischen Bildgebung vorgesehenen Röntgenstreumessvorrichtung ist in Fig. 3 dargestellt. Wie bereits erwähnt, muss der Tunnel 30 geschlossene, gegen Strahlung abgeschirmte Seitenwände aufweisen. Für Patientenmessungen ist es von Vorteil, den Querschnitt des Tunnels 30 möglichst groß einzurichten, wobei man jedoch von dem Grundsatz, dass alle Voxel des Durchstrahlungsbereichs messaktiv sein sollen, nicht abweichen will. Fig. 3 zeigt deshalb einen Messaufbau analog zu Fig. 1, wobei der Tunnel ein gleichschenkliges Trapez als Querschnitt aufweist. Die absoluten vertikalen Lagen von Unter- und Oberseite des Tunnels 30 sind apparativ vorbestimmt. Dies legt zugleich die Geometrie der verschiedenen Fächer von Fingerstrahlen zwischen Unter- und Oberseite des Tunnels 30 fest. Die Trapez-Seitenwände sind nun ausgerichtet entlang des Verlaufs der äußersten Röntgen-Fingerstrahlen, die bei Aktivierung einer der beiden äußeren Röntgenanoden emittiert werden, zwischen Trapez-Unterseite und Trapez-Oberseite. Damit schließt das gleichschenklige Trapez den größtmöglichen Durchstrahlungsbereich ein, der ausschließlich messaktive Voxel umfasst.

Es kann angemerkt werden, dass ein trapezförmiger Querschnitt des Tunnels 30 für Gepäck-Scanner, die sich auf ein Förderband zum Transport der Gepäckstücke durch den Durchstrahlungsbereich verlassen, eher ungeeignet ist. Die Gepäckstücke könnten sich an den abgeschrägten Seitenwänden verkeilen oder von diesen gedreht oder umgeworfen werden. Eine Patientenmessung ist in dieser Hinsicht jedoch unkritisch und wird überdies ohnehin von einem Operateur überwacht.

Für die Auflageplattform 60 ist wenigstens ein Antriebsmittel vorzusehen, dass die Auflageplattform 60 kontrolliert durch den Tunnel 30 entlang der Tunnelachse bewegen kann. Unter einer kontrollierten Bewegung der Auflageplattform 60 ist im Kontext der Erfindung zu verstehen, dass die relative Lage der Auflageplattform 60 zum Tunnel 30 zu jedem Zeitpunkt während einer Messung genau bekannt ist und dass auch jede vorbestimmte relative Lage durch Steuerbefehle des Operateurs an das wenigstens eine Antriebsmittel herbeigeführt werden kann.

Dies ist vorzugsweise dadurch zu erreichen, dass die Röntgenstreumessvorrichtung mit Tunnel 30 und die Auflageplattform 60 mit dem wenigstens einen Antriebsmittel eine bauliche Einheit bilden und die Auflageplattform 60 relativ zum Tunnel 30 überhaupt nur durch die Ansteuerung des wenigstens einen Antriebsmittels, beispielsweise eines elektrischen Schrittmotors, bewegt werden kann.

Alternativ kann auch eine zeitweise Entkopplung der Auflageplattform 60 von dem wenigstens einen Antriebsmittel zur händischen Bewegung der Auflageplattform 60 vorgesehen sein, wenn die Auflageplattform (60) mit Hilfsmitteln ausgestattet ist, um ihre Lage vor Beginn einer Röntgenstreumessung präzise zu ermitteln. Solche Hilfsmittel können beispielsweise optische Markierungen oder Retroreflektoren sein, die an vorbestimmten Stellen der Auflageplattform angeordnet sind und von einer optischen Detektorzeile erfasst werden, wenn sie diese passieren.

Es können auch weitere Antriebsmittel vorgesehen sein, die die seitlichen Versatzbewegungen unabhängig von der Vorschubbewegung durch den Durchstrahlungsbereich kontrollieren. Alternativ kann ein einzelnes Antriebsmittel, insbesondere ein Elektromotor, zur Durchführung der Vorschub- und der Versatzbewegung genutzt werden, wobei ein schaltbares Getriebe dazu ausgebildet ist, den Kraftangriff des Antriebsmittels nacheinander in verschiedene Abtriebe einzuspeisen, welche Bewegungen der Auflageplattform (60) in Vorschubrichtung oder in wenigstens eine Versatzrichtung vollführen können. Es ist für die Zwecke der Erfindung ausreichend, wenn zu jedem Zeitpunkt nur eine Bewegung entlang genau einer der genannten Richtungen erfolgen kann.

Im Folgenden sollen Verfahrensschritte zur Verwendung der erfindungsgemäßen Röntgenstreumessvorrichtung zur medizinischen Bildgebung erläutert werden. Die Verfahrensschritte sind mittels herkömmlicher programmierbarer Recheneinheiten und elektronischer Ansteuerung in Hardware implementierbar und können zur Ausgestaltung der Vorrichtung herangezogen werden.

In erster Linie ist die Röntgenstreumessvorrichtung dazu ausgebildet und vorgesehen, einen Patienten über zueinander parallel verlaufende gerade Pfade entlang der Achse des Tunnels (30) durch den Durchstrahlungsbereich zu bewegen. Dies wird vorzugsweise dadurch erreicht, dass das wenigstens eine Antriebsmittel veranlasst wird, wenigstens eine erste Bewegung der Auflageplattform (60) über eine vorbestimmte Vorschubstrecke entlang der Tunnelachse, hiernach eine zweite Bewegung über eine vorbestimmte Versatzstrecke senkrecht zur Tunnelachse und hiernach eine dritte Bewegung über die Vorschubstrecke durchzuführen. Dabei spielt es keine Rolle, ob die dritte Bewegung der ersten entgegengerichtet abläuft oder nicht, solange sie nur über dieselbe Vorschubstrecke wie die erste erfolgt.

Während jeder Bewegung der Auflageplattform 60 über die Vorschubstrecke kann eine Röntgenstreumessung durchgeführt werden, wobei die während der Bewegung erfassten Röntgenstreumessdaten in ein Impulsübertrags-Profil bezogen auf Voxel mit einer vorbestimmten Voxel-Kantenlänge in einem Objektkoordinatensystem umgerechnet und als ein 4D-Datensatz bereitgestellt werden. Somit können aus den vorgenannten drei Bewegungen zwei 4D-Datensätze bestimmt und bereitgestellt werden, die denselben Patienten gescannt über dieselbe Vorschubstrecke zeigen, wobei alle Datenunterschiede auf die Versatzstrecke zurückzuführen sind. Besonders vorteilhaft ist es, wenn die Länge der Versatzstrecke kleiner als die Voxel-Kantenlänge entlang der Versatzrichtung vorbestimmt wird. Zwei derart erzeugte 4D-Datensätze können mit einem Super-Resolutions-Verfahren verarbeitet werden, um einen verfeinerten, d.h. höher aufgelösten 4D-Datensatz zu errechnen.

Die Super-Resolution beschreibt allgemein ein Verfahren zur Erhöhung der bildlichen Auflösung einer Aufnahme. Hierbei werden mehrfache Aufnahmen desselben Objektes gemacht, die durch Pixel oder Sub-Pixel zueinander versetzt sind. Durch die Interpolation auf ein feineres Grid bei Kenntnis des Versatzes können die Aufnahme kombiniert und das Ergebnis dadurch verbessert werden. Es wird auch auf das Lehrbuch von Chaudhuri, Subhasis, ed. "Super-resolution imaging", Vol. 632. Springer Science & Business Media, 2001 verwiesen.

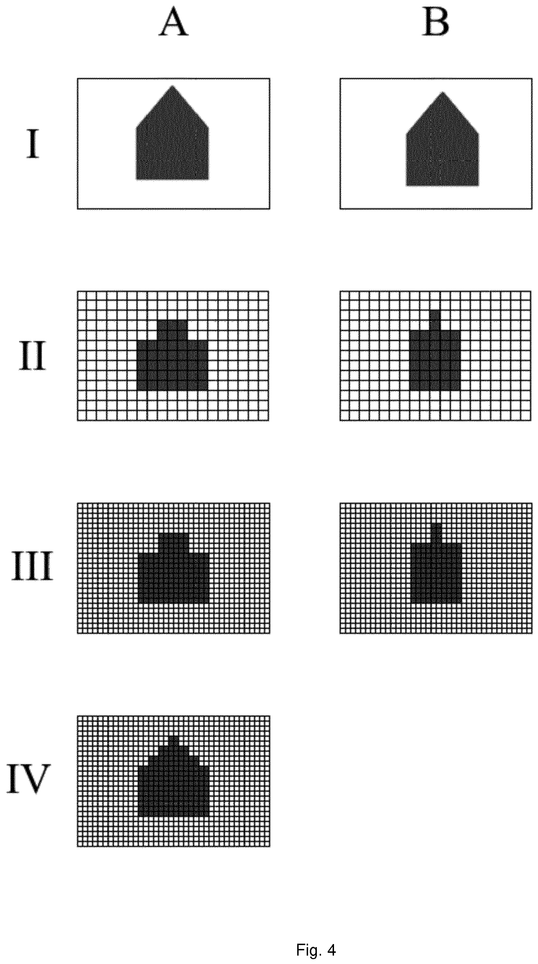

Zur Verdeutlichung skizziert Fig. 4 eine mögliche Vorgehensweise. Dasselbe Objekt wird zweimal mit einem ortsauflösenden Detektor - hier: Digitalkamera - erfasst (Zeile I), wobei die Erfassung einmal ohne (Spalte A) und einmal mit (Spalte B) einem seitlichen Versatz erfolgt. Die auf den Detektorpixeln erfassten Messwerte sind in Zeile II angedeutet, wobei die Pixel-Kantenlängen relativ groß sind und jeweils "verpixelte" Datensätze entstehen. In Zeile III sind die Messdaten auf ein verfeinertes Grid mit einer verkleinerten Pixel-Kantenlänge, die etwa der halben bisherigen Pixel-Kantenlänge entspricht, übertragen worden. Ein Super-Resolutions-Verfahren setzt in Zeile III an und analysiert die Messdaten der beiden Grids (A) und (B) auf die in der Zusammenschau enthaltene Information, die sog. Transinformation, und weist den Pixeln hiernach interpolierte Werte zu, die zu einer Maximierung der Transinformation führen. Das so erzielte Resultat, nämlich ein verfeinertes Abbild des Objekts, ist in Zeile IV dargestellt. Übergänge und Kantenverläufe sind darin deutlich besser zu erkennen als in den ursprünglich erfassten Messdaten.

Auf die Einzelheiten der Messung und Maximierung der Transinformation kommt es in der vorliegenden Erfindung nicht an. Es genügt festzuhalten, dass sich Super-Resolutions-Verfahren ohne weiteres von Pixel auf Voxel übertragen lassen, und dass auch Messwerte, die nicht wie bei Bildern Grau- oder Farbwerte sind, sondern Funktionen über einem eindimensionalem Raum, beispielsweise hier die Impulsübertrags-Profile, einer Messung der Transinformation zugänglich sind.

Nach den vorangegangenen Gesichtspunkten ist es somit vorteilhaft, dass aus einem ersten 4D-Datensatz aus den Messdaten der ersten Bewegung der Auflageplattform und aus einem zweiten 4D-Datensatz aus den Messdaten der dritten Bewegung mittels eines Superresolutions-Verfahrens ein Impulsübertrags-Profil bezogen auf Voxel mit einer gegenüber den Ausgangsdatensätzen verkleinerten Voxel-Kantenlänge interpoliert wird.

Dabei ist es zweckmäßig, die Voxel in Objektkoordinaten möglichst gleichmäßig in drei Dimensionen zu verkleinern, beispielsweise jeweils auf die halbe Kantenlänge. Dies kann exemplarisch dadurch erreicht werden, dass die Längen der Versatzstrecken zu ΔX = dX / 2 und ΔY = dY / 2 gesetzt werden, wobei dX und dY wie bereits zuvor die apparativ vorbestimmten Voxel-Kantenlängen des Durchstrahlungsbereichs bezeichnen. Die Voxel-Kantenlänge in Richtung der Tunnelachse ist durch die Wahl der Vorschubgeschwindigkeit V_{P} sehr einfach veränderbar über dZ = V_{P} * T_{M}. Die Halbierung von V_{P} führt unmittelbar zur Halbierung von dZ, ohne dass dies einer Interpolation bedarf.

Insgesamt muss ein Patient also wenigstens dreimal durch den Durchstrahlungsbereich der Röntgenstreumessvorrichtung bewegt werden, wenn man eine Auflösungsverbesserung in drei Dimensionen erreichen möchte, nämlich einmal ohne seitlichen Versatz und je einmal mit einem Versatz um die Streckenlänge ΔX oder ΔY. Da die Vorschubgeschwindigkeit zugleich bei allen drei Bewegungen gleich und verringert ist, erhöht sich die Strahlungsdosis des Patienten gegenüber einer Einzelmessung, nämlich im Beispiel um den Faktor sechs, wenn alle Voxel-Kantenlängen halbiert werden sollen.

Die Gesamtdosis ist auch bei mehrfachen Messungen immer noch geringer als bei einem Volumen CT, das dieselbe Auflösung nativ erzeugen kann.

Die mit Hilfe der Erfindung erzielbare Auflösungsverbesserung gegenüber Röntgenstreumessvorrichtungen nach dem Stand der Technik gestattet die Erzeugung medizinisch verwertbarer Datensätze, die für einen Arzt interpretierbare, bestimmten anatomischen Gegebenheiten zuzuordnende Informationen enthalten. Wie bereits erläutert, sind die Messergebnisse als 4D-Datensätze nicht direkt verständlich, sondern sie zeigen chemische Zusammensetzungen an und benötigen eine Vorauswertung. Vorzugsweise erfolgt diese dadurch, dass aus dem 4D-Datensatz eines lebenden Patienten anhand des Vergleichs mit tabellierten Daten für vorbekannte Gewebe je Voxel eine Gewebezusammensetzung ermittelt wird. Solche Tabellen lassen sich beispielsweise anhand extrahierter und histologisch bestimmter Gewebeproben erstellen, aber auch lebendes in situ Gewebe sollte dabei einfließen.

Der besondere Vorteil einer medizinischen Bildgebung mit Röntgenstreumessungen liegt in der potenziell hohen Aussagekraft in Bezug auf Gewebeveränderungen, die sich gerade erst anbahnen, noch nicht unbedingt symptomatische Auswirkungen haben und nicht mit anderen bildgebenden Modalitäten abbildbar sind. Ihre mögliche Vorhersagefähigkeit macht diese Technologie interessant, um frühzeitig Maßnahmen gegen entstehende Krankheiten einleiten zu können.

Zu diesem Zweck sind mehrfache Wiederholungen der Messungen an einem Patienten in größeren Zeitabständen sehr empfehlenswert, um gezielt nach Unterschieden in den chemischen Verhältnissen des Patientenkörpers zu suchen. Konkret wird also vorgeschlagen, dass aus einer Mehrzahl von verschiedenen 4D-Datensätzen desselben Patienten unter Berücksichtigung vorbestimmter Signifikanzschwellenwerte Unterschiede in den Gewebezusammensetzungen je Voxel ermittelt werden. Die Signifikanzschwellenwerte sind dabei zu verstehen als Intervallgrenzen, in denen Gewebezusammensetzungen im Zuge üblicher Prozesse variieren ohne Anlass zur Besorgnis zu geben. Diese werden für verschiedene Gewebe, insbesondere innere Organe, unterschiedlich ausfallen und können beim Erstellen der vorgenannten Tabellen mit ermittelt und ebendort eingetragen werden. Ziel solcher Schwellenwerte soll es sein, die Aufmerksamkeit des Arztes auf Veränderungen zu lenken, die nicht in den "gesunden" Rahmen fallen, und somit signifikant für potenzielle Erkrankungen sein können.

Für die Vergleichbarkeit von 4D-Datensätzen aus zeitlich deutlich getrennten Messungen ist es von großer Bedeutung, dieselben Körperpartien des Patienten aus beiden Messungen einander korrekt zuzuordnen, da sich ansonsten schon aus den Lageänderungen erhebliche Unterschiede der Gewebezusammensetzung ergeben würden. Es ist a priori aber nicht auszuschließen, dass der Patient bei der zweiten Messung tatsächlich nicht genauso auf der Patientenliege liegt wie bei der ersten. Beispielsweise könnte er Schmerzen haben, die ihn eine unbewusste Schonhaltung einnehmen lassen. Ebenso könnte er erheblich an Gewicht gewonnen oder verloren haben.

Die mit größter Wahrscheinlichkeit zwischen den Messungen unveränderte Gewebestruktur des Patienten ist indes sein Skelett. Da sich die Streumessdaten von Knochen von denen des Weichgewebes gut unterscheiden lassen und die Knochen - bei Erwachsenen - als praktisch forminvariant angenommen werden können, kann man das Skelett als patientenimmanenten Referenzrahmen verwenden. Insbesondere erscheint es vorteilhaft, dass die Gesamtheit aller Voxel eines 4D-Datensatzen, denen ein Knochengewebe des Patienten zugeordnet ist, als eine Skelett-Untermenge von Voxeln selektiert wird. Diese Skelett-Untermenge gilt es dann, in beiden 4D-Datensätzen aufzufinden und in eine Korrespondenz zu bringen.

Es bietet sich insbesondere an, die Skelett-Untermengen zweier 4D-Datensätze zur Deckung zu bringen, d.h. die 4D-Datensätze derart in ein gemeinsames Koordinatensystem einzutragen, dass die dreidimensionalen Positionen der Knochengewebe enthaltenden Voxel übereinstimmen. Dabei ist es selbstverständlich wichtig, dass die räumliche Nachbarschaftsstruktur aller Voxel eines 4D-Datensatzes, die Topologie, erhalten bleiben.

Es wird deshalb als vorteilhaft angesehen, dass auf wenigstens einen einer Mehrzahl von verschiedenen 4D-Datensätzen desselben Patienten eine Topologie erhaltende Transformation angewandt wird, so dass die Skelett-Untermengen wenigstens zweier 4D-Datensätze in ihren Voxelpositionen übereinstimmen.

Solche Topologie erhaltende Transformationen sind aus der numerischen Mathematik hinreichend bekannt. Sie kommen z.B. dort zur Anwendung, wo der Einfluss von Krafteinwirkungen auf deformierbare Objekte simuliert wird, und basieren beispielweise auf einer Finite-Elemente-Methode (FEM). Andere Anwendungen von Topologie erhaltenden Transformationen liegen in der Computer Vision und betreffen beispielsweise die Gestensteuerung, wo numerische Skelett-Modelle mit hoher Geschwindigkeit in kamerabasierte Messwerte eingepasst werden müssen. Die dabei ermittelten Verschiebungsvektoren für Knotenpunkte des Skelettmodells erhalten im Idealfall die Anordnung und Länge der Knotenverbindungen (Kanten) und lassen sich im Wege der Interpolation auf umgebenden Volumina bzw. Voxel übertragen.

Abschließend soll darauf hingewiesen werden, dass die vorbeschriebenen Verfahrensschritte zur Auswertung von erfassten Röntgenstreumessdaten vollständig in einem herkömmlichen Personal Computer ausgeführt werden können, der nicht zwangsläufig in die erfindungsgemäße Röntgenstreumessvorrichtung integriert sein muss. Vielmehr können die vom Detektor-Array (50) erfassten Messwerte zusammen mit Daten, die das Rückschließen auf die Positionen der Auflageplattform 60 während des Entstehens der Messwerte gestatten, zunächst einfach in einen nichtflüchtigen Datenspeicher abgelegt werden. Die Datenauswertung kann im Nachgang, z.B. durch Entnahme und Transport des Datenspeichers zu einem PC oder durch Herstellen einer Kommunikationsverbindung zwischen Datenspeicher und PC, mit Hilfe einer separaten Auswertesoftware erfolgen, die auf den oben genannten Verfahrensschritten basiert.

Es wird jedoch als sachgerecht angesehen, einen derart in die Messvorrichtung integrierten Rechner zur Auswertung vorzusehen, dass die Auswertung unverzüglich nach der Messung und vollautomatisch erfolgt, so dass der behandelnde Arzt ggf. noch in Anwesenheit des Patienten beliebige Schnittbilder zur Beurteilung der Gesundheitslage des Patienten zur Verfügung gestellt bekommen kann.

## Patentansprüche

1. Röntgenstreumessvorrichtung aufweisend einen Tunnel (30) zur Aufnahme eines Objekts und einen vorbestimmten Durchstrahlungsbereich im Innern des Tunnels (30), der mit einer Mehrzahl von Fächern aus Röntgen-Fingerstrahlen durchsetzbar ist, sowie eine in den Tunnel (30) einbringbare Auflageplattform (60) für das Objekt mit wenigstens einem Antriebsmittel zur kontrollierten Bewegung der Auflageplattform (60) im Tunnel (30), **dadurch gekennzeichnet, dass** die Auflageplattform (60) mit dem wenigstens einen Antriebsmittel zur Bewegung entlang der Tunnelachse und entlang wenigstens einer Achse senkrecht zur Tunnelachse ausgebildet ist.

2. Röntgenstreumessvorrichtung nach Anspruch 1, **dadurch gekennzeichnet, dass** die Auflageplattform (60) rechteckig ausgebildet ist, wobei die langen Seiten entlang der Tunnelachse orientiert sind.

3. Röntgenstreumessvorrichtung nach Anspruch 2, **dadurch gekennzeichnet, dass** wenigstens an den langen Seiten Seitengeländer (70) gebildet aus einem Metall oder aus einem Kunststoffkomposit mit einer vom Patientenkörper unterscheidbaren Röntgensignatur angeordnet sind.

4. Röntgenstreumessvorrichtung nach einem der vorangehenden Ansprüche, **dadurch gekennzeichnet, dass** der Durchstrahlungsbereich eine gegen die Lotrichtung in Richtung der Tunnelachse geneigte Ebene ist.

5. Röntgenstreumessvorrichtung nach einem der vorangehenden Ansprüche, **dadurch gekennzeichnet, dass** die Auflageplattform (60) wenigstens auf ihrer Oberseite muldenförmig ausgebildet ist.

6. Röntgenstreumessvorrichtung nach einem der vorangehenden Ansprüche, **dadurch gekennzeichnet, dass** der Tunnel (30) ein gleichschenkliges Trapez als Querschnittsfläche aufweist, wobei die Trapez-Seitenwände entlang des Verlaufs der äußersten Röntgen-Fingerstrahlen zwischen Trapez-Unterseite und Trapez-Oberseite ausgerichtet sind.

7. Verfahren zur Röntgenstreumessung mit einer Vorrichtung nach einem der vorangehenden Ansprüche, **dadurch gekennzeichnet, dass** das wenigstens eine Antriebsmittel veranlasst wird, wenigstens eine erste Bewegung der Auflageplattform (60) über eine vorbestimmte Vorschubstrecke entlang der Tunnelachse, hiernach eine zweite Bewegung über eine vorbestimmte Versatzstrecke senkrecht zur Tunnelachse und hiernach eine dritte Bewegung über die Vorschubstrecke durchzuführen.

8. Verfahren nach Anspruch 7, **dadurch gekennzeichnet, dass** die während der Bewegung der Auflageplattform (60) über die Vorschubstrecke erfassten Röntgenstreumessdaten in ein Impulsübertrags-Profil bezogen auf Voxel mit einer vorbestimmten Voxel-Kantenlänge in einem Objektkoordinatensystem umgerechnet und als ein 4D-Datensatz bereitgestellt werden.

9. Verfahren nach Anspruch 8, **dadurch gekennzeichnet, dass** die Länge der Versatzstrecke kleiner als die Voxel-Kantenlänge entlang der Versatzrichtung vorbestimmt wird.

10. Verfahren nach Anspruch 9, **dadurch gekennzeichnet, dass** aus einem ersten 4D-Datensatz aus den Messdaten der ersten Bewegung der Auflageplattform und aus einem zweiten 4D-Datensatz aus den Messdaten der dritten Bewegung mittels eines Superresolutions-Verfahrens ein Impulsübertrags-Profil bezogen auf Voxel mit einer gegenüber den Ausgangsdatensätzen verkleinerten Voxel-Kantenlänge interpoliert wird.

11. Verfahren zur medizinischen Röntgenstreumessung nach einem der Ansprüche 7 bis 10, **dadurch gekennzeichnet, dass** aus dem 4D-Datensatz eines lebenden Patienten anhand des Vergleichs mit tabellierten Daten für vorbekannte Gewebe je Voxel eine Gewebezusammensetzung ermittelt wird.

12. Verfahren nach Anspruch 11, **dadurch gekennzeichnet, dass** aus einer Mehrzahl von verschiedenen 4D-Datensätzen desselben Patienten unter Berücksichtigung vorbestimmter Signifikanzschwellenwerte Unterschiede in den Gewebezusammensetzungen je Voxel ermittelt werden.

13. Verfahren nach einem der Ansprüche 11 oder 12, **dadurch gekennzeichnet, dass** die Gesamtheit aller Voxel eines 4D-Datensatzen, denen ein Knochengewebe des Patienten zugeordnet ist, als eine Skelett-Untermenge von Voxeln selektiert wird.

14. Verfahren nach Anspruch 13, **dadurch gekennzeichnet, dass** auf wenigstens einen einer Mehrzahl von verschiedenen 4D-Datensätzen desselben Patienten eine Topologie erhaltende Transformation angewandt wird, so dass die Skelett-Untermengen wenigstens zweier 4D-Datensätze in ihren Voxelpositionen übereinstimmen.
